# EUROPÄISCHE PATENTSCHRIFT

(11) **EP 0 749 759 B1**
(45) Veröffentlichungstag und Bekanntmachung des Hinweises auf die Patenterteilung: **30.01.2002**
(21) Anmeldenummer: 96108132.0
(22) Anmeldetag: 22.05.1996
(51) Int. Cl.: A61M 5/28, A61M 5/315

(54) **Vorrichtung zur Abgabe eines fliessfähigen Stoffes aus einem Behälter**
Device for dispensing a fluid from a receptacle
Dispositif de distribution d'une substance fluide à partir d'un réceptacle

(30) Priorität: 21.06.1995 DE 19522451
(43) Veröffentlichungstag der Anmeldung: 27.12.1996
(73) Patentinhaber: Hansen, Bernd, Dipl.-Ing., D-74429 Sulzbach-Laufen (DE)
(72) Erfinder: Hansen, Bernd, 74429 Sulzbach-Laufen (DE); Leu, Willy, 5057 Reitnau (CH)
(74) Vertreter: Patentanwälte Bartels und Partner

(56) Entgegenhaltungen:
- EP-A- 0 047 398
- WO-A-83/00882
- DE-A- 4 234 383
- DE-A- 4 419 235
- DE-B- 1 219 632
- FR-A- 2 174 705

## Beschreibung

Die Erfindung betrifft eine Vorrichtung zur Abgabe eines fließfähigen Stoffes aus einem aus Kunststoff bestehenden, deformierbaren Behälter gemäß den Merkmalen des Oberbegriffes des Anspruches 1.

Eine gattungsgemäße Vorrichtung ist durch die EP-A-47 398 bekannt. Die bekannte Lösung ist als mehrteiliger Spritzenkörper aufgebaut, wobei ein von Hand betätigbarer Spritzenkolben einen mit einem Wirkstoff befüllten Behälter bodenseitig innerhalb einer topfförmigen Buchse verfährt, bis dieser für eine vollständige Entnahme an den Schultern des Spritzenkörpers anliegt, die in Verlängerung vorstehende Griffe für die Betätigung der Spritze aufweisen. Mit Abziehen des Kopfes wird eine umhüllte Spritzennadel freigegeben, die innerhalb des Halses geführt bei Gebrauch die Behältniswand für eine Wirkstoffabgabe durchstößt. Die bekannte Vorrichtung dient insbesondere dem Einsatz von Spritzen bei Notfällen und steht unmittelbar für eine Wirkstoffabgabe bereit. Eine genaue Dosierung des Wirkstoffes mit der bekannten Abgabevorrichtung ist nicht erreichbar und aufgrund der Teilevielfalt ist die bekannte Lösung teuer in der Herstellung. Des weiteren ist die Sterilität nicht in dem gewünschten Maße erreichbar.

Durch die DE-A-4 234 383 ist eine medizinische Spritze zur Aufnahme und Abgabe von Flüssigkeiten bekannt, bei welcher diese durch Druckabgabe über einen eigenständigen Modul aufgezogen bzw. herausgepreßt werden, wobei sämtliche mit der Flüssigkeit in Berührung kommenden Bereiche der Spritze als ein- oder mehrteiliger Modul mit nur geringem Werkstoffeinsatz gestaltet sind und nach der Applikation der Flüssigkeit bedienungsfreundlich entnommen und einem Recycling zugeführt werden können. Dabei ist der äußere Modul der Spritze durch die Auswechslung des inneren Moduls vielfach wiederverwendbar ohne weitere Reinigung. Die dahingehende Lösung ist zwar umweltgerecht, genügt aber hohen Anforderungen an die Sterilität nicht und eine genaue Dosierung des abzugebenden Stoffes ist mit dieser bekannten Lösung gleichfalls nicht möglich.

Ausgehend von diesem Stand der Technik liegt der Erfindung die Aufgabe zugrunde, die Abgabe des im Behälter enthaltenen Stoffes genau zu dosieren, die Herstellkosten niedrig zu halten bei Gewährleistung eines hohen Maßes an Sterilität. Eine dahingehende Aufgabe löst eine Vorrichtung mit den Merkmalen des Anspruches 1.

Dadurch, daß gemäß dem kennzeichnenden Teil des Anspruches 1 an den Hals unter Bildung eines Brechverschlusses der Kopf angeformt ist und daß der Körper, der Hals und der Kopf einstückig ausgebildet sind, ist der Behälter im Blasform- oder Saugformverfahren kostengünstig herstellbar, wobei der Behälter in der Form gefüllt und verschlossen wird, so daß sich die erfindungsgemäße Vorrichtung auch für solche Stoffe eignet, die unter sterilen Bedingungen abgefüllt und bis zum Gebrauch aufbewahrt werden müssen.

Vor der Entnahme des im Behälter enthaltenen Stoffes wird der Kopf längs der Sollbruchstelle vom Ansatz des Halses abgetrennt und die Vorrichtung beispielsweise in Form einer Einwegspritze ist unmittelbar gebrauchsfertig und die Sterilität bis zum dahingehenden Trennvorgang gewährleistet. Dadurch, daß der Benutzer den Behälter nicht unmittelbar mit den Fingern zusammendrücken muß, ihm vielmehr hierfür eine den Behälter aufnehmende Buchse und ein in dieser verschiebbarer Kolben sowie eine dessen Verschiebeweg in der Buchse erkennbar machende Anzeigeeinrichtung zur Verfügung steht, kann der Behälter ohne Schwierigkeiten in demjenigen Maße in seiner Länge reduziert werden, das notwendig ist, um die gewünschte Menge des im Behälter enthaltenen Stoffes abzugeben. Dabei kann jeder Behälter im verkaufsfertigen Zustand in der zugeordneten Buchse bereits enthalten sein. Man kann aber auch den Behälter erst vor einer Abgabe seines Inhaltes in die Buchse einführen und die Buchse sowie den zugehörigen Kolben für eine Mehrzahl gleichartiger Behälter verwenden.

In den Hals und/oder den Kopf kann ein ein- oder mehrteiliger Einlegekörper eingelegt sein. Vorzugsweise erfolgt das Einlegen vor der endgültigen Formung des den Einlegekörper aufnehmenden Teiles des Behälters, also während sich der Behälter noch in der Herstellungsform befindet. Bei dem Einlegekörper kann es sich beispielsweise um einen Stopfen handeln. Besonders vorteilhaft ist ein Einlegekörper, der eine Injektionsnadel trägt, die erst beim Abtrennen des Kopfes freigelegt wird. Der Behälter kann dann zusammen mit der erfindungsgemäßen Vorrichtung als Einwegspritze verwendet werden. Eine derartige Einwegspritze ist äußerst kostengünstig.

Bei einer bevorzugten Ausführungsform bestehen sowohl die Buchse als auch der Kolben aus Kunststoff, weil sie dann in einfacher und kostengünstiger Weise hergestellt werden können.

Die Anzeigeeinrichtung kann eine Skala und eine mit ihr zusammenwirkende Marke aufweisen. Beispielsweise kann die Skala dann, wenn der Behälter durchsichtig ist, auf diesen vorgesehen und die Marke am Kolben vorgesehen sein, wobei auch das im Inneren der Buchse liegende Ende des Kolbens die Marke bilden kann. Ebenso ist es aber auch möglich, die Skala am Kolben vorzusehen und die Buchse mit einer Marke zu versehen oder deren offenes Ende als Marke zu verwenden. Die Anzeigeeinrichtung kann aber auch zusammenwirkende Rastelemente aufweisen, mittels deren der Kolben in der Buchse in Positionen kraftschlüssig verrastbar ist, die längs seines Verschiebeweges, vorzugsweise in gleichen Abständen, vorgesehen sind. Diese Rastelemente sind vorzugsweise an die Buchse bzw. den Kolben angeformt. Beispielsweise kann der Kolben mit einem Vorsprung und die Buchse mit Vertiefungen oder umgekehrt versehen sein. Zweckmäßigerweise haben die Vertiefungen die Form von Rillen, damit es keine Ausrichtschwierigkeiten gibt.

Um sicherzustellen, daß nur eine sehr geringe Restmenge im Behälter enthalten ist, wenn dieser vollständig zusammengedrückt ist, wird zweckmäßigerweise der Hals des Behälters so kurz wie möglich gemacht. Ferner kann der Kolben an dem am Körper anliegenden Ende eine axial vorspringende Materialpartie aufweisen, welche den Behälterboden in das Behälterinnere drückt.

Um die erfindungsgemäße Vorrichtung bequem handhaben zu können, ist bei einer bevorzugten Ausführungsform an der Buchse ein von ihrer Außenseite abstehender Griff vorgesehen, der beispielsweise eine Anlagefläche für den Zeigefinger und den Mittelfinger bildet, damit nur noch der Daumen zur Betätigung des Kolben benötigt wird. Dieser Griff kann beispielsweise aus einem an die Buchse angeformten Flansch oder zwei diametral an ihr angeordneten Armen bestehen.

Daß auch gegen Ende der Abgabe die Vorrichtung noch gut gehandhabt werden kann, ist die Länge des Kolbens bei einer vorteilhaften Ausführungsform mindestens gleich der Länge der Buchse.

Für den Behälter kommen unterschiedliche Formen in Frage, beispielsweise kann ein im Vergleich zur axialen Länge großer Außendurchmesser gewählt werden, so daß sich die den Mantel des Behälters bildende Wand nicht besonders stark zu deformieren braucht. Vorteilhaft ist aber auch ein Behälter in Form eines Faltenbalges.

Im folgenden ist die Erfindung an Hand von der Zeichnung dargestellten Ausführungsbeispielen im einzelnen erläutert. Es zeigen
- Fig. 1: eine teilweise in Längsrichtung geschnitten dargestellte Seitenansicht eines ersten Ausführungsbeispiels im Zustand vor dem Gebrauch,
- Fig. 2: eine teilweise in Längsrichtung geschnitten dargestellte Ansicht des ersten Ausführungsbeispiel im vollständig entleerten Zustand,
- Fig. 3: einen unvollständig dargestellten Längsschnitt durch den Kopf und den Hals des Behälters gemäß den Fig. 1 und 2 bei abgetrenntem Kopf,
- Fig. 4: eine teilweise in Längsrichtung geschnitten dargestellte Ansicht eines zweiten Ausführungsbeispiels im Zustand vor dem Gebrauch,
- Fig. 5: eine teilweise in Längsrichtung geschnitten dargestellte Seitenansicht eines dritten Ausführungsbeispiels vor dem Gebrauch,
- Fig. 6: eine Ansicht entsprechend Fig. 5 des dritten Ausführungsbeispiels nach dem Öffnen des Behältnisses und vor der Abgabe seines Inhaltes,
- Fig. 7: eine Ansicht entsprechend Fig. 5 des dritten Ausführungsbeispiels im vollständig entleerten Zustand,
- Fig. 8: eine teilweise in Längsrichtung geschnitten dargestellte Ansicht eines vierten Ausführungsbeispiels.

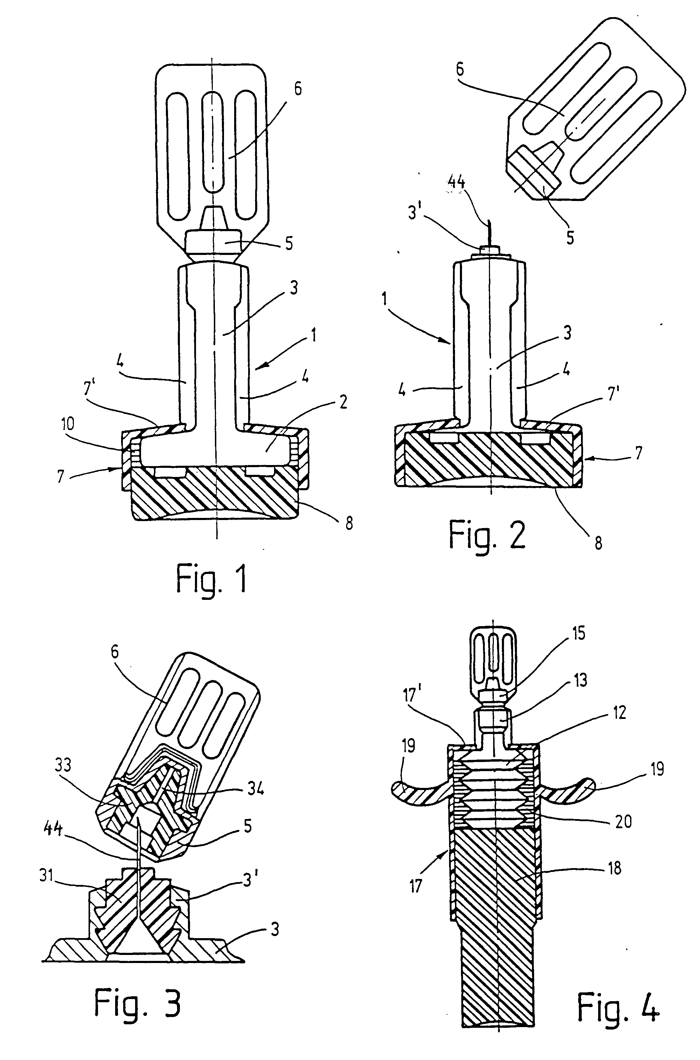

Das in den Fig. 1 und 2 in vergrößertem Maßstab dargestellte erste Ausführungsbeispiel weist ein aus Kunststoff bestehendes Behältnis 1 auf, das wegen seines geringen Volumens auch als Ampulle bezeichnet werden kann. Das Behältnis 1 besteht aus einem Körper 2, dessen Durchmesser um ein Mehrfaches größer ist als seine axiale Länge, einem sich an den Körper 2 anschließenden Hals 3, der durch zwei angeformte und diametral angeordnete Rippen 4, die sich über die gesamte Länge des Halses 3 erstrecken, versteift ist, und einem Kopf 5, welcher den Hals 3 an dem vom Körper 2 wegweisenden Ende verschließt. Zwischen einem im Durchmesser reduzierten Ansatz 3' des Halses 3 und dem Kopf 5 ist eine Einschnürung mit reduzierter Wandstärke zur Bildung eines Brechverschlusses vorgesehen. Um ohne Schwierigkeiten die für das Abbrechen des Kopfes 5 vom Hals 3 erforderliche Kraft auf den Kopf 5 ausüben zu können, ist an diesem ein flacher Griffteil 6 angeformt. Der Durchmesser des Körpers 2 ist um ein Mehrfaches größer als der Durchmesser des Halses 3. Die den Übergang von der Mantelfläche des Körpers 2 zum Hals 3 bildende Schulterfläche ist praktisch neigungsfrei. Ferner ist die Dicke der die Mantelfläche des Körpers 2 bildenden Wand wesentlich geringer als diejenige des Bodens und der diesem gegenüberliegenden Schulterfläche. Die Mantelfläche des Körpers 2 kann deshalb mit relativ geringer Kraft so weit verformt werden, daß der Boden in Anlage an den ihm gegenüberliegende Schulterfläche kommt.

Der Behälter 1 wird vorzugsweise gemäß dem in der DE 44 39 231 A1 beschriebenen Blasformverfahren hergestellt, in der Blasform luftfrei und steril gefüllt und außerdem in der Blasform durch das Anformen des Kopfes 5 verschlossen. Vor dem Formen des Ansatzes 3', der Einschnürung und des Kopfes 5 sowie des Griffteiles 6' kann ein Einlegekörper eingesetzt werden.

Im Ausführungsbeispiel ist in den Ansatz 3' des Halses 3 ein Nadelträger 31 mit einer Injektionsnadel 44 eingesetzt, die, wie Fig. 3 zeigt, über das dem Hals 3 abgewandte Ende des Nadelträgers 31 übersteht. Auf dieses Ende ist ein Schutzkörper 33 aufgesetzt, der in den Kopf 5 eingesetzt ist und in einem Kanal 34 die Injektionsnadel 44 aufnimmt. Wenn der Kopf 5 längs der Bruchstelle des Brechverschlusses vom Ansatz 3' abgetrennt wird, wird der fest mit dem Kopf 5 verbundene Schutzkörper 33 von dem fest mit dem Ansatz 3' verbundenen Nadelträger 31 abgehoben.

Das Behältnis 1 ist im verkaufsfertigen Zustand oder wird, ehe die eingefüllte Flüssigkeit, bei der es sich im Ausführungsbeispiel um ein zu injizierendes pharmazeutisches Präparat handelt, entnommen wird, in eine topfförmige Buchse 7 eingesetzt, in deren Boden 7' eine zentrale Öffnung für den Durchtritt des Kopfes 5 und des Halses 3 vorgesehen ist. In die formstabile, als Kunststoffspritzteil ausgebildete Buchse 7 greift ein ebenfalls formstabiler, als Kunststoffspritzteil ausgebildeter Kolben 8 ein, der längsverschiebbar in der Buchse 7 geführt ist und im Ausführungsbeispiel eine axiale Länge hat, die gleich der axialen Länge des Körpers 2 ist.

Die Buchse 7 besteht im Ausführungsbeispiel aus einem durchsichtigen Material und ist in ihrer Mantelfläche mit einer in axialer Richtung verlaufenden Skala versehen, die entsprechend der Mindestmenge bei der dosierten Abgabe unterteilt ist. Mit dieser Skala wirkt die umlaufende Kante des am Boden des Körpers 2 anliegenden Endes des Kolbens 8 zusammen.

Vor einer Entnahme des im Behälter 1 enthaltenen Stoffes wird der Kopf 5 längs der Sollbruchstelle vom Ansatz 3' des Halses 3 abgetrennt, indem der Griffteil 6 relativ zum Hals 3 gedreht oder gekippt wird. Nunmehr ist die Einwegspritze gebrauchsfertig. Nach dem Einführen der Injektionsnadel 32 kann der Kolben 8 entsprechend dem abzugebenden Volumen in die Buchse 7 hineingedrückt werden, wobei die Volumenänderung und damit auch die abgegebene Menge an der Skala der Buchse 7 in Verbindung mit der Lage der Kante des Kolbens 8 abgelesen werden kann.

Das Hineindrücken des Kolbens 8 in die Buchse 7 kann mit einer Hand erfolgen, indem der Hals 3 zwischen den Zeigefinger und den Mittelfinger genommen und mit dem Daumen auf das zunächst aus der Buchse 7 herausragende Ende des Kolbens 8 gedrückt wird. Das Behältnis 1 ist bis auf das im Hals 3 befindende Restvolumen vollständig entleert, wenn der Boden des Körpers 2 an dem Übergang zum Hals 3 anliegt, wie Fig. 2 zeigt.

Zusätzlich zu der Skala oder statt dieser kann man die Buchse 7 oder den Kolben 8 mit einer Reihe von ringnutartigen Vertiefungen versehen, in welche ein ringnutartiger Wulst, der an den anderen Teil angeformt ist, kraftschlüssig einrastet. Es können dann dosiert Mengen des Stoffes abgegeben werden, welche der Volumenänderung zwischen zwei aufeinander folgenden Rillen entsprechen. Im Ausführungsbeispiel ist die Innenmantelfläche des Körpers 2 mit in axialer Richtung nebeneinander angeordneten, ringförmigen Rillen 10 versehen, in die ein Ringwulst des Kolbens 8 einrastet.

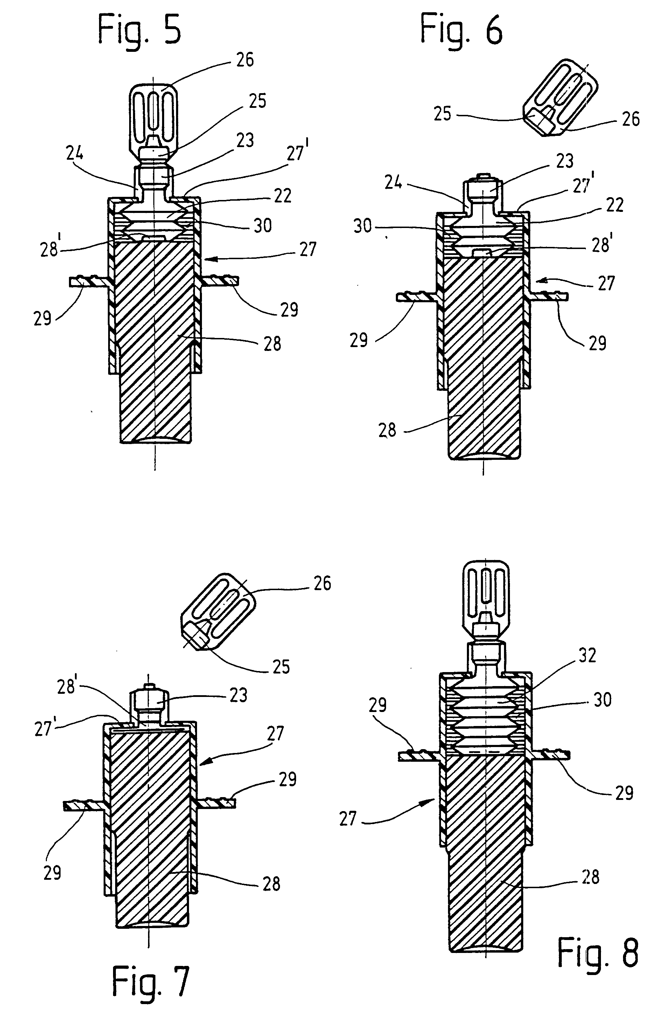

Die in Fig. 4 dargestellte Ausführungsform der erfindungsgemäßen Vorrichtung weist einen Behälter auf, dessen Körper 12 die Form eines Faltenbalges hat. Ein solcher Körper kann ebenfalls im Blasformverfahren hergestellt werden. An den in seiner Längsrichtung zusammendrückbaren, aus Kunststoff bestehenden Körper 12 ist ein Hals 13 angeformt, dessen Länge wesentlich geringer ist als bei dem Ausführungsbeispiel gemäß den Fig. 1 bis 3, um die im Hals 13 verbleibende Restmenge so klein wie möglich zu halten. Über eine eingeschnürte Sollbruchstelle ist an den Hals 13 ein Kopf 15 angeformt. Das Herstellen des Behälters 1, das Einfüllen des Stoffes und das Verschließen kann wie bei dem ersten Ausführungsbeispiel im Blasformverfahren unter sterilen Bedingungen erfolgen. Ferner kann, wie dort, ein ein- oder mehrteiliger Einlegekörper in den Hals 13 oder einen Ansatz desselben eingelegt sein, von dem sich ein Teil im Kopf 15 befinden kann.

Der Körper 12 ist oder wird in einer topfförmigen Buchse 17 angeordnet, deren Boden 17' eine zentrale Durchtrittsöffnung für den Hals 13 hat. Der Innendurchmesser der Buchse 17 ist wie derjenige der Buchse 7 wenigstens soviel größer als der Außendurchmesser des Körpers 12 bzw. 2 vor einer Längenreduzierung, als dessen Durchmesser bei der Längenreduzierung größer wird. In der als Kunststoffspritzteil ausgeführten Buchse 17 ist ein ebenfalls als Kunststoffspritzteil ausgeführter Kolben 18 längsverschiebbar geführt. Da im Ausführungsbeispiel am Boden 17' der Buchse 17 keine ausreichend große Anlagefläche für zwei Finger vorhanden ist, sind außen an die Buchse 17 zwei diametral abstehende Arme 19 angeformt, auf die zwei Finger aufgelegt werden können, während mit dem Daumen der Kolben 18 für eine dosierte Abgabe des im Behälter enthaltenen Stoffes kontrolliert in die Buchse 7 hineingrdrückt und dabei der Körper 12 entsprechend in seiner Länge reduziert wird.

Wie bei dem ersten Ausführungsbeispiel ist auch beim zweiten Ausführungsbeispiel eine in Fig. 4 nicht sichtbare Anzeigeeinrichtung für den Verschiebeweg des Kolbens in der Buchse vorgesehen. Sofern diese Anzeigeeinrichtung eine Skala aufweist, kann diese am Kolben 18 oder an der Buchse 17 vorgesehen sein. Mit ihr wirkt dann eine Markierung am anderen Teil zusammen. Man kann aber auch bei diesem Ausführungsbeispiel statt einer Skala oder zusätzlich zu dieser an der Buchse 17 und dem Kolben 18 Rastelemente vorsehen, beispielsweise in der Innenfläche der Buchse 17 eine Reihe von ringförmigen Rillen 20, in welche ein an den Kolben 18 angeformter Vorsprung kraftschlüssig einrasten kann.

Das in den Fig. 5 bis 7 dargestellte dritte Ausführungsbeispiel unterscheidet sich von dem zweiten Ausführungsbeispiel außer dem kleineren Volumen und damit einer kleineren axialen Länge des Körpers 22 seines Behälters nur dadurch, daß außen an die Buchse 27 als Griffteil ein radial abstehender Flansch 29 angeformt ist. Der Hals 23, der Kopf 25 und dessen Griffteil 26 sind wie bei dem zweiten Ausführungsbeispiel ausgebildet, weshalb wegen weiterer Einzelheiten auf die Ausführungen zu letzteren Bezug genommen wird. Auch dieses Ausführungsbeispiel kann als Einwegspritze ausgebildet sein.

Ehe eine dosierte Abgabe des im Körper 22 enthaltenen Stoffes erfolgt, wird zunächst der Kopf 25 längs der Sollbruchstelle vom Hals 23 abgetrennt. Dann wird der Kolben 28 mit Hilfe der Anzeigeeinrichtung kontrolliert in die Buchse 27 gedrückt, wobei sich die axiale Länge des Körpers 22 entsprechend reduziert. Die Anzeigeeinrichtung weist nebeneinander angeordnete, ringförmige Rillen 30 in der Innenmantelfläche der Buchse 27 und einen Ringwulst am Kolben 28 auf. Ein axialer Vorsprung 28' des Kolbens 28 an den am Boden des Körpers 22 anliegenden Ende stellt sicher, daß bei vollständiger Zusammendrückung des Körpers 22 nur noch eine äußerst geringe Restmenge des Stoffes im Hals 23 enthalten ist, zumal der Vorsprung 28' noch etwas in den Hals 23 eindringt, wie Fig. 7 zeigt. Wenn der Körper 22 vollständig entleert ist, steht im Ausführungsbeispiel der Kolben 22 noch etwas über das offene Ende der Buchse 27 über. Außerdem ist, wie Fig. 7 ebenfalls zeigt, der Kolben 28 im Anschluß an den an der Innenwand der Buchse 27 anliegenden Abschnitt in seinem Durchmesser etwas reduziert, um trotz eines reibungsbehaftenden Sitzes den Kolben 28 mit relativ geringer Kraft in der Buchse 27 verschieben zu können.

Die Abmessungen der Buchse 27 und des Kolbens 28 sind so gewählt, daß, wie Fig. 8 zeigt, die Buchse 27 auch einen Behälter aufnehmen kann, dessen Körper 32 wesentlich länger ist als der Körper 22. Die Buchse 27 und der Kolben 28 sind wie bei den zuvor beschriebenen Ausführungsbeispielen Kunststoffspritzteile, die mit einer die kontrollierte Verschiebung des Kolbens erkennbar machende Anzeige versehen sind. Sofern ein Einlegekörper mit einer Injektionsnadel eingelegt ist, kann auch dieses Ausführungsbeispiel als Einwegspritze verwendet werden.

## Patentansprüche

1. Vorrichtung zur Abgabe eines fließfähigen Stoffes aus einem aus Kunststoff bestehenden, deformierbaren Behälter (1), der einen in seiner Längsrichtung zusammendrückbaren Körper (2,12,22,32) und einen sich an dessen eines Ende unter Bildung einer Schulter anschließenden Hals (3,13,23) kleineren Durchmessers aufweist, durch den hindurch entsprechend der bei der Längenreduzierung des Körpers (2,12,22,32) sich ergebenden Volumenverminderung die Abgabe erfolgt, wobei für eine Dosierung des aus dem Hals (3,13,23) austretenden Stoffes
a) eine topfförmige Buchse (7,17,27), die in ihrem an die Schulter des Körpers (2,12,22,32) anlegbaren Boden (7',17',27') eine Durchtrittsöffnung für den Hals (3,13,23) aufweist, der von einem Kopf (5,15,25) abschließbar ist,
b) ein Kolben (8,18,28), der in der Buchse (7,17,27) gegen deren Boden (7',17',27') hin verschiebbar ist, und
c) eine den Verschiebeweg des Kolbens (8,18,28) in der Buchse (7,17,27) erkennbar machende Anzeigeeinrichtung vorgesehen sind,
**dadurch gekennzeichnet, daß** an den Hals (3,13,23) unter Bildung eines Brechverschlusses der Kopf (5,15,25) angeformt ist und daß
der Körper (2,12,22,32), der Hals (3,13,23) und der Kopf (5,15,25) einstückig ausgebildet sind.

2. Vorrichtung nach Anspruch 1, **dadurch gekennzeichnet, daß** sowohl die Buchse (7,17,27) als auch der Kolben (8,18,28) aus Kunststoff bestehen.

3. Vorrichtung nach Anspruch 1 oder 2, **dadurch gekennzeichnet, daß** die Anzeigeeinrichtung eine Skala und eine mit dieser zusammenwirkende Marke aufweist.

4. Vorrichtung nach einem der Ansprüche 1 bis 3, **dadurch gekennzeichnet, daß** die Anzeigeeinrichtung zusammenwirkende Rastelemente (10,20,30) aufweist, mittels deren der Kolben (8,18,28) in der Buchse (7,17,27) in Positionen kraftschlüssig verrastbar ist, die längs seines Verschiebewege in vorzugsweise gleichen Abständen voneinander vorgesehen sind.

5. Vorrichtung nach Anspruch 4, **dadurch gekennzeichnet, daß** die Rastelemente aus einer Reihe von Vertiefungen (10; 20; 30) und wengistens einem Rastvorsprung bestehen, die in den aneinander anliegenden Mantelflächen der Buchse (7; 17; 27) und des Kolbens (8; 18; 28) vorgesehen sind.

6. Vorrichtung nach einem der Ansprüche 1 bis 5, **dadurch gekennzeichnet, daß** der Kolben (28) an dem am Körper (22) anliegenden Ende eine axial vorspringende Materialpartie (28') aufweist.

7. Vorrichtung nach einem der Ansprüche 1 bis 6, **gekennzeichnet durch** einen von der Außenseite der Buchse (17; 27) abstehenden Griff (19; 29).

8. Vorrichtung nach Anspruch 7, **dadurch gekennzeichnet, daß** der Griff aus einem an die Buchse (17; 27) angeformten Flansch (29) oder zwei diametral angeordneten Armen (19) besteht.

9. Vorrichtung nach einem der Ansprüche 1 bis 8, **dadurch gekennzeichnet, daß** die Länge des Kolben (8; 18; 28) mindenstens gleich der Länge der Buchse (7; 17; 27) ist.

10. Vorrichtung nach einem der Ansprüche 1 bis 9, **dadurch gekennzeichnet, daß** der Körper (12; 22; 32) des Behälters die Form eines Faltenbalges hat.

11. Vorrichtung nach einem der Ansprüche 1 bis 10, **dadurch gekennzeichnet, daß** der Hals (3, 3') einen Einlegkörper (31) enthält.

12. Vorrichtung nach Anspruch 11, **dadurch gekennzeichnet, daß** für eine Verwendung als Einwegspritze der Einlegekörper (31) eine Injektionsnadel (44) trägt.

## Claims

1. Device for dispensing a fluid from a deformable plastic container (1), comprising a body (2, 12, 22, 32) which may be compressed in its longitudinal direction, providing a smaller diameter in a neck (3, 13, 23) formed at one end of the container where a shoulder is formed, through which the fluid is dispensed when the volume inside the container is reduced through the longitudinal reduction of the body (2, 12, 22, 32), whereby the following is provided for metering the fluid expelled from the neck (3, 13, 23), namely
a) a droplet-shaped bush (7, 17, 27), through the bottom (7', 17', 27') adjacent to the shoulder of the body (2, 12, 22, 32) of which extends a port for the neck (3, 13, 23) which can be sealed by a head (5, 15, 25),
b) a piston (8, 18, 28), slidably arranged against the bottom (7', 17', 27') of the bush (7, 17, 27), and
c) an indicator, indicating the slide-way of the piston (8, 18, 28) inside the bush (7, 17, 27),
**characterised in that** the head (5, 15, 25) is formed on the neck (3, 13, 23) thereby creating a breakable closure and that
the body (2, 12, 22, 32), the neck (3, 13, 23) and the head (5, 15, 25) are formed as a single piece.

2. Device according to claim 1, **characterised in that** the bush (7, 17, 27) and the piston (8, 18, 28) are made from plastic.

3. Device according to claims 1 or 2, **characterised in that** the indicator provides a scale and a marker indicating on the scale.

4. Device according to one of the claims 1 to 3, **characterised in that** the means for indication provides co-operating locking elements (10, 20, 30) with which the piston (8, 18, 28) can be gravity locked inside the bush (7, 17, 27) in positions, which are provided along the slide-way, preferably in equal distances.

5. Device according to claim 4, **characterised in that** the locking elements comprise indentations (10, 20, 30) and at least one locking protrusion, which are provided in the casing surfaces of the bush (7, 17, 27) and the piston (8, 18, 28).

6. Device according to one of the claims 1 to 5, **characterised in that** the piston (28) at the side adjacent to the body (22) provides an axially protruding material piece (28').

7. Device according to one of the claims 1 to 6, **characterised by** a handle (19, 29) positioned at a distance from the exterior of the bush (17, 27).

8. Device according to claim 7, **characterised in that** the handle comprises a flange (29) formed onto the bush (17, 27) or two diametrically arranged arms (19).

9. Device according to one of the claims 1 to 8, **characterised in that** the length of the piston (8, 18, 28) is at least equal to that of the bush (7, 17, 27).

10. Device according to one of the claims 1 to 9, **characterised in that** the body (12, 22, 32) of the container takes the form of a bellow.

11. Device according to one of the claims 1 to 10, **characterised in that** the neck (3, 3') contains a body within (31).

12. Device according to claim 11, **characterised in that** the body within (31), when used as non-re-usable syringe, is fitted with an injection needle (44).

## Revendications

1. Dispositif pour délivrer un produit fluide à partir d'un conteneur déformable (1) réalisé en matière synthétique, qui comprend un corps (2, 12, 22, 32) compressible dans le sens de sa longueur et un col (3, 13, 23) d'un diamètre plus petit, qui se rattache à l'une des extrémités du corps en formant un épaulement, et à travers lequel a lieu l'évacuation du produit en fonction de la réduction de volume résultant de la diminution de la longueur du corps (2, 12, 22, 32),
a) une douille en forme de pot (7, 17, 27), qui comprend dans son fond (7', 17', 27') pouvant être appliqué sur l'épaulement du corps (2, 12, 22, 32) une ouverture de passage pour le col (3, 13, 23), lequel peut être coiffé d'une tête (5, 15, 25),
b) un piston (8, 18, 28), qui peut être déplacé dans la douille (7, 17, 27) contre le fond (7', 17', 27') de celle-ci et
c) un dispositif indicateur rendant visible le trajet de déplacement du piston (8, 18, 28) dans la douille (7, 17, 27)
étant prévus pour le dosage du produit sortant du col (3, 13, 23), **caractérisé en ce que** la tête (5, 15, 25) est formée sur le col (3, 13, 23) en constituant un élément de rupture et **en ce que** le corps (2, 12, 22, 32), le col (3, 13, 23) et la tête (5, 15, 25) sont réalisés d'une seule pièce.

2. Dispositif selon la revendication 1, **caractérisé en ce qu'**aussi bien la douille (7, 17, 27) que le piston (8, 18, 28) sont réalisés en matériau synthétique.

3. Dispositif selon la revendication 1 ou 2, **caractérisé en ce que** le dispositif indicateur est constitué d'une échelle graduée et d'un repère de visualisation fonctionnant en interaction avec celle-ci.

4. Dispositif selon l'une des revendications 1 à 3, **caractérisé en ce que** le dispositif indicateur comprend des éléments de crantage interactifs (10, 20, 30), au moyen desquels le piston (8, 18, 28) peut être bloqué dans la douille (7, 17, 27) dans des positions, qui sont prévues le long de son trajet de déplacement et qui présentent de préférence les mêmes espacements.

5. Dispositif selon la revendication 4, **caractérisé en ce que** les éléments de crantage se composent d'une série de creux (10, 20, 30) et d'au moins une saillie crantée, qui sont prévus dans les surfaces latérales de la douille (7, 17, 27) et du piston (8, 18, 28).

6. Dispositif selon l'une des revendications 1 à 5, **caractérisé en ce qu'**à son extrémité s'appuyant sur le corps (22) le piston (28) comprend une partie de matériau dépassant en saillie axiale (28').

7. Dispositif selon l'une des revendications 1 à 6, **caractérisé en ce que** le côté extérieur de la douille (17, 27) est muni d'une poignée (19, 29).

8. Dispositif selon la revendication 7, **caractérisé en ce que** la poignée se compose d'une collerette (29) formée avec la douille (17, 27) ou de deux bras disposés dans des positions diamétralement opposées (19).

9. Dispositif selon l'une des revendications 1 à 8, **caractérisé en ce que** la longueur du piston (8, 18, 28) est au moins égale à la longueur de la douille (7, 17, 27).

10. Dispositif selon l'une des revendications 1 à 9, **caractérisé en ce que** le corps (12, 22, 32) du conteneur a la forme d'un soufflet.

11. Dispositif selon l'une des revendications 1 à 10, **caractérisé en ce que** le col (3, 3') contient un insert (31).

12. Dispositif selon la revendication 11, **caractérisé en ce que** pour une utilisation comme seringue jetable, l'insert (31) est muni d'une aiguille d'injection (44).
